(19) Europäisches Patentamt
European Patent Office
Office européen des brevets

(11) EP 1 653 930 B1

(12) **EUROPEAN PATENT SPECIFICATION**

(45) Date of publication and mention of the grant of the patent:
**19.12.2007 Bulletin 2007/51**

(21) Application number: **04738465.6**

(22) Date of filing: **05.08.2004**

(51) Int Cl.:
***A61K 9/20*** *(2006.01)* ***A61K 31/40*** *(2006.01)*

(86) International application number:
**PCT/CZ2004/000045**

(87) International publication number:
**WO 2005/011638 (10.02.2005 Gazette 2005/06)**

(54) **METHODS FOR THE STABILIZATION OF ATORVASTATIN**

VERFAHREN ZUR STABILISIERUNG VON ATORVASTATIN

PROCEDES DE STABILISATION DE L'ATORVASTATINE

(84) Designated Contracting States:
**AT BE BG CH CY CZ DE DK EE ES FI FR GB GR HU IE IT LI LU MC NL PL PT RO SE SI SK TR**
Designated Extension States:
**LT LV**

(30) Priority: **05.08.2003 CZ 20032135**
**03.08.2004 CZ 20040857**

(43) Date of publication of application:
**10.05.2006 Bulletin 2006/19**

(73) Proprietor: **Zentiva, a.s.**
**102 37 Praha 10 (CZ)**

(72) Inventors:
- **SEBEK, Pavel**
  **161 00 Praha 6 (CZ)**
- **PROKOPOVA, Alena**
  **101 00 Praha 10 (CZ)**
- **SVOBODA, Eduard**
  **102 00 Praha 10 (CZ)**
- **RADL, Stanislav**
  **143 00 Praha 2 (CZ)**
- **STACH, Jan**
  **190 00 Praha 9 - Ujezd nad Lesy (CZ)**
- **SVOBODA, Martin**
  **553 41 Lazne Bohdanec (CZ)**
- **KOVACIK, Andrej**
  **920 01 Hlohovec (SK)**
- **DANKO, Adrian**
  **920 01 Hlohovec (SK)**
- **PETRO, Roman**
  **920 01 Hlohovec (SK)**
- **STRIZINEC, Miroslav**
  **920 01 Hlohovec (SK)**

(74) Representative: **Jirotkova, Ivana et al**
**Rott, Ruzicka & Guttmann,**
**Nad Stolou 12**
**170 00 Praha 7 (CZ)**

(56) References cited:
**WO-A-01/93859**
**WO-A-20/04032920**
**US-A1- 2003 042 166**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 653 930 B1

## Description

Technical Field

This invention relates to stabilization of the extremely unstable substance atorvastatin, in its crystalline, but particularly amorphous state.

Background Art

The hemicalcium salt of (*3R,5R*) 7-[3-phenyl-4-phenylcarbamoyl-2-(4-fluorophenyl)-5-isopropylpyrrol-1-yl]-3,5-dihydroxyheptanoic acid of formula I

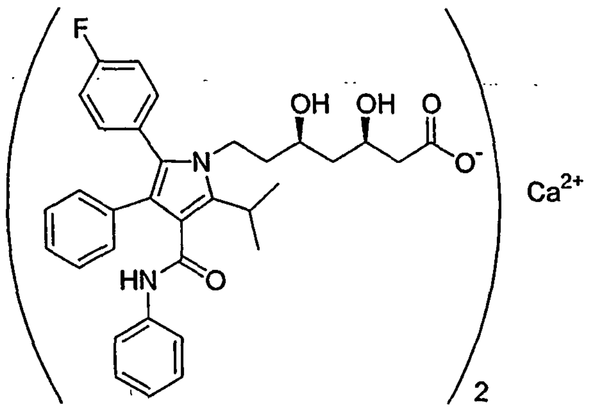

(I)

known under the non-proprietary name atorvastatin (I), in the text sometimes called the calcium salt of atorvastatin, is produced according to published patents (US patents 4,681,893 and 5,273,995). The said drug is an important representative of hypolipidemic and hypocholesteric drugs.

Atorvastatin can exist in various crystalline forms or in an amorphous form. The preparation of various polymorphs is described in published patents (US 5,969,156; US 6,121,461; WO 03/004470 and WO 01/36384), the amorphous form in patent US 6,087,511. The crystalline forms are, according to the above-mentioned patents, much more stable than the amorphous form.

Also the authors of patent EP 680320 pointed out that the substance atorvastatin has insufficient stability. It is stated in the specification of the said patent that it is an unstable substance sensitive to heat, humidity, low pH of the environment, and light, particularly UV radiation. A composition whose main feature are basic inorganic substances is the solution to this problem. Hydroxides, oxides or carbonates are the preferred anions. As to cations, most often calcium, magnesium, and lithium salts are stated. Calcium carbonate is stated as the best solution. Antioxidants of anisole or ascorbate type are also added to the recommended composition.

In WO 00/34525, the stabilization of a dosage form is solved by adding buffers, especially citrates.

WO 01/76566 solves the stabilization of a dosage form by adding a basic polymer containing amino or amido groups, for example polyvinylpyrrolidone.

WO 01/93859 solves the stabilization of HMG-CoA inhibitors, and among them also of atorvastatin, using a substance capable of binding and neutralizing carbon dioxide. Carbon dioxide is, according to the authors of the application, the most important factor leading to the instability of the product. Its effect is ascribed to the lowering of pH, which results in the decomposition of hydroxyacids particularly to their lactones. It is pointed out that gastric troubles may be caused if a medicine with a high content of alkaline substances is administered to patients. This fact limits the possibility of improving the stability by adding a stabilizer to the dosage form.

WO 02/072073 shows the relation between the $pK_a$ value of atorvastatin and the pH value of an aqueous solution of a solid dosage form. According to the quoted application, the dosage form should contain such ingredients which would cause the pH of solution to reach a value not lower than $pK_a + 1$.

Accordingly, it follows from the prior art that the main methods how to solve the problem of the stability of atorvastatin in a dosage form were either increase of the pH of the dosage form, or prevention of the lowering of the pH by $CO_2$ contained in the atmosphere.

Despite these measures, the dosage forms of atorvastatin, and particularly if amorphous atorvastatin is in these forms, showed significant instability. Although the formation of undesirable products such as the lactone of atorvastatin

was prevented, the formation of other unknown substances occurred. The active substance itself, not in the dosage form, showed even worse stability. Therefore, it was necessary to store and transport amorphous atorvastatin at about -20 °C. Naturally, these measures increased the costs of the said operations.

In addition to the above-mentioned methods, a new method for the preservation of substances susceptible to oxidation with the use of substances trapping air oxygen, often called oxygen absorbers, has been developed. Mitsubishi Gas Chemical (Tokyo, Japan) have developed bags absorbing oxygen based on a reaction of iron under the trade name Ageless (Yoshikawa, Y., Amemiya, A.; Komatsu, T.; Inoue, Y.; Yuyama, M., Oxygen Absorbent for Food Packaging. Jpn. Kokai Tokkyo Koho, Showa 56-33980, 1978). Similar products are also offered, for example, by Multisorb Technologies, Inc. under the trade name Fresh Pax™ or by Standa Industry under the trade name ATCO.

Many products are available nowadays. They are based on humidity-activated oxygen absorbers, self-activating absorbers, ultraviolet-radiation-activated absorbers, radiation-activated absorbers, microwaves-activated absorbers, absorbers activated by a combination of activation processes, or absorbers not requiring any activation.

In patent application US 2002/0132359, the use of these absorbers for pharmaceutical preparations sensitive to oxygen is applied for protection. The application is carried out in a blister packing where the absorber is situated between the lid and the blister itself. The application further informs that it is very difficult to find out which of the substances will be susceptible to oxidation. The problem subsists in the fact that often the oxidation does not follow the classical Arrhenius equation, and that is why accelerated stability tests, which are successfully used for other decomposition reactions, fail. The patent application further contains a list of some pharmaceutical substances, which could be sensitive to oxygen. HMG-CoA inhibitors simvastatin or lovastatin are the most relevant ones among them. Both these substances contain a system of conjugated double bonds in a carbocyclic system, which can result in sensitivity to oxygen.

Patent application WO 2004/032920 discloses a pharmaceutical formulation with an amorphous active substance, i.e. atorvastatin calcium, exposed to an inert gas atmosphere. In the examples vials and blisters are disclosed comprising tablets with atorvastatin calcium in atmosphere of nitrogen or argon with minimal oxygen content. Minimal increase of impurities has been achieved already in the atmosphere with 2.4% of oxygen. It did not seem that further oxygen decreasing to 0.4% caused further decomposition decrease.

One of frequently discussed impurities is atorvastatin lactone of formula II

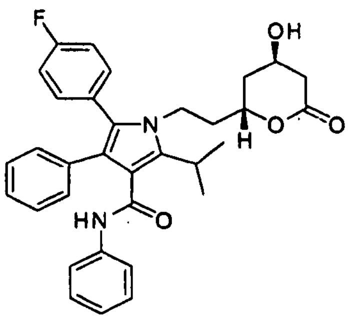

(II)

Lactonization is an acid-catalyzed process proceeding probably via the free dihydroxyacid of atorvastatin. Therefore, a solution subsists in adding basic substances to the dosage forms.

Disclosure of the Invention

This invention consists in a method for the stabilization of the pharmaceutical active solid substance atorvastatin embedded in a gaseous mixture.
characterized in that a drug in the form of tablets or capsules containing atorvastatin in an amount of 1 to 60 % by weight, packaged in a blister is stabilized and in the surrounding gaseous mixture a partial pressure of oxygen of at most 2 kPa is maintained.

Detailed Description of the Invention:

We have shown, with the help of exactly controlled experiments, that the instability is also caused by oxidation by atmospheric oxygen. Particularly amorphous atorvastatin shows significant instability towards oxidation, whereas crystalline forms are somewhat more stable. However, this is caused by statistical factors because a substance firmly incorporated in a crystal lattice has a lesser probability to react with atmospheric oxygen than a substance in an amorphous form (Stephen R. Byrn: Solid State Chemistry of Drugs, Academic Press, 1982).

Therefore, the oxidative decomposition of crystalline forms of atorvastatin is slower than that of the amorphous form. This is also important for dosage forms because, for example, mechanical stress when producing tablets may lead to partial collapse of the crystalline structure causing instability of the dosage form.
The following experiments were carried out in order to determine the instability of atorvastatin. The purpose was to find out which factors lead to the degradation of the product.

1. Set of stress tests. The stock solution of atorvastatin (2 ml) was gradually subjected to the following experiments:

a. boil (24 hr) with 2 ml of 0.2 N hydrochloric acid,
b. boil (24 hr) with 2 ml of 0.2 N acetic acid,
c. boil (24 hr) with 2 ml of 0.2 N sodium hydroxide,
d. boil (24-hr) with 2 ml of 4% hydrogen peroxide,
e. boil (24 hr) with 2 ml of water,
f. irradiation with UV radiation (5 hr),
g. irradiation with visible light (24 hr),
h. solid substance heated at 100 °C for 24 hr,
i. solid substance subjected to UV radiation (5 hr),
j. solid substance subjected to visible light (24 hr).

The results of analytical assessment (HPLC) are summarized in the following table (Table 1):

Table 1

| Conditions | Content of Atorvastatin, % |
|---|---|
| 24 hr boil in 0.1 N HCl | 2.1 |
| 24 hr boil in 0.1 N AcOH | 83.2 |
| 24 hr boil in 0.1 N NaOH | 72.0 |
| 24 hr boil in 2% $H_2O_2$ | 54.8 |
| 24 hr boil in water | 88.4 |
| 5 hr under UV in water | 64.3 |
| 24 hr in the light | 104.5 |
| substance 24 hr, 100 °C | 96.3 |
| substance 5 hr, UV | 99.3 |
| substance 24 hr, light | 96.5 |

What could particularly be seen from the results was instability in the acid environment. Furthermore, the substance decomposed significantly also in a solution when subjected to UV radiation, which is in conformity with literary data (Tetrahedron 49,10,1979-1984,1993).
Decomposition by hydrogen peroxide turned out to be another significant factor.
In order to make the carried-out experiments more precise, stability tests of solid amorphous atorvastatin and of several selected dosage forms were established. As to containers, polyethylene (PE) and an aluminium foil with a sealable PE layer (Al + PE) were used. Some of the stability data were determined in nitrogen atmosphere ($N_2$) (with a partial pressure of oxygen of roughly 3 kPa).
The results of these stability tests are summarized in Table 2.

Table 2

| Time, months | Temp., °C | Package | $P_o$, kPa | Impurities, % |
|---|---|---|---|---|
| 0 | - | entry | - | 0.21 |
| 3 | 5 | 2x RE | 18 | 0.39 |
| 3 | 5 | PE+A1 | 18 | 0.34 |
| 3 | 5 | PE+A1 | 3 | 0.34 |

(continued)

| Time, months | Temp., °C | Package | $P_O$, kPa | Impurities, % |
|---|---|---|---|---|
| 3 | 25 | 2x PE | 18 | 0.77 |
| 3 | 25 | PE+A1 | 18 | 0.63 |
| 3 | 25 | PE+A1 | 3 | 0.43 |
| 6 | 5 | 2x PE | 18 | 0.83 |
| 6 | 5 | PE+A1 | 18 | 0.71 |
| 6 | 5 | PE+A1 | 3 | 0.44 |

It is obvious from the table that at 25 °C, the content of impurities already after 3 months markedly depends on the partial pressure of oxygen in the package. At 5 °C this dependency manifests more markedly only after 6 months. Also the effect of the manner of packaging on the final content of impurities can be seen from the table. The substance in an air-tight PE+A1 package shows abetter stability than in permeable PE bags.

In order to precisely determine the decomposition mechanism, the following experiments examining only the oxidative decomposition of atorvastatin were carried out. A recent publication /Pharmaceutical Development and Technology, 7(1), 1-32 (2002)/ described a set of experiments which are suitable for the recognition of oxidation of substances and for the determination of its mechanism.

The following experiments were carried out:

a. oxidation of a 1% solution of atorvastatin in the system of ethyl acetate - acetonitrile (1:1) at 40 °C using a radical initiator (2,2'-azobiscyanopentanoic acid) at a pressure of 1 MPa of oxygen;
b. oxidation of a 1% solution of atorvastatin in the system of ethyl acetate - acetonitrile (1:1) at 40 °C without a radical initiator;
c. control experiment in the system of ethyl acetate - acetonitrile (1:1) at 40 °C using a radical initiator (2,2'-azobiscyanopentanoic acid) in an inert atmosphere of argon (the partial pressure of oxygen found as being about 1 kPa).

The results are summarized in Table 3.

Table 3

| Time, hours | Initiator | $P_O$, MPa | Impurities, % |
|---|---|---|---|
| 0 | | entry sample | 0.49 |
| 24 | yes | 1 | 6.42 |
| 48 | yes | 1 | 24.37 |
| 72 | yes | 1 | 30.93 |
| 24 | yes | 0.001 | 0.98 |
| 48 | yes | 0.001 | 1.38 |
| 24 | no | 1 | 9.27 |

It follows from the results that atmospheric oxygen itself can oxidize atorvastatin and no radical initiator is necessary for the oxidation. The control experiment in an inert atmosphere of argon showed a small increase in the amount of the lactone of atorvastatin caused by increased temperature and slightly acidic radical initiator.

The comparison of the profile of impurities from stability tests (Table 2) with the profile of impurities created by oxidation using HPLC-MS was another result of the experiment. It has shown that all significant impurities the amount of which increased during the stability tests, with the exception of the lactone of atorvastatin, are formed by oxidation. On the basis of this knowledge of oxidative decomposition, a solution was looked for which would prevent the contact of the substance itself or the substance in a dosage form with atmospheric oxygen. According to our; and also in literature published /K.C. Waterman; M.C. Roy: Pharmaceutical Development and Technology, 7 (2), 227-234 (2002)/, experience, it is very difficult, when packing the dosage form, to reach the partial pressure of oxygen lower than 2 - 3 kPa without using a vacuum step. This is, however, a sufficient amount to cause oxidation of the product still occur. An especially suitable method how to achieve a lower concentration of oxygen (as low as below the value of 0.1 kPa) is the use of

either vacuum or oxygen absorbers. Therefore, further experiments were carried out with the use of vacuum or of oxygen absorbers. The experiments were carried out with oxygen absorbers Ageless™ from Mitsubishi Gas Chemical and ATCO from Standa Industry. Many other commercially available absorbers can also be advantageously used; for example FreshPax™ (Multisorb Technologies), O-Buster™ (Hsiao Sung Non-Oxygen Chemical Co), Biotika Oxygen Absorber (Biotika) and the like.

Other aspects of the invention include packaging under nitrogen or argon by a newly developed method. As cited above, pharmaceutical packaging under nitrogen made in the usual manner into a blister does not achieve a partial pressure lower than 2-3 kPa. These values are not sufficient in the case of an especially sensitive substance like atorvastatin.

In our below described method of packaging, values of the partial pressure of oxygen lower than 1 kPa, in a preferred embodiment lower than 0.4 kPa, can be achieved in a usual pharmaceutical blister.

The above-mentioned results show that oxidative degradation is an important factor mainly for amorphous atorvastatin and this fact has to be taken into consideration when storing the substance or the final dosage form. We have shown that the use of oxygen absorbers significantly improves the storability of amorphous atorvastatin (examples 1 and 2). It clearly follows from the results that the protection of atorvastatin from atmospheric oxygen completely prevents its decomposition. When using oxygen absorbers, the substance can be then stored at 25 °C without any limitations, which means, in comparison with the storage at a lower temperature, a significant decrease of costs. The substance can also be stored in other containers which let oxygen through partially with the final concentration of oxygen lower than 1 %, ideally lower than 0.1 %. The substance can also be advantageously packed in an inert atmosphere, which makes the lifetime of the oxygen absorber longer and the initial exothermic reaction when trapping oxygen by the absorption bag milder. The needed capacity of the absorption bag and the resultant equilibrium concentration of oxygen (in ppm) can be calculated from the following equation (Vinod Daniel, Frank L. Lambert: Waac Newsletter 15, 2, 1993, 12-14, 1993):

$$[O_2] = L / 12.7\ C$$

wherein L is the leakage rate of oxygen out of the container in ppm/day, C is absorbance, which is the ratio of the capacity of the absorption bag and the total volume of the container.

The stabilization method of this invention relates both to the active substance itself and the dosage forms containing atorvastatin, especially atorvastatin in the amorphous state. The described dosage forms of atorvastatin contain 1 to 60 % by weight, preferably 3 to 20 % by weight of the active substance and several auxiliary substances with various functions, especially to help to release the active substance in a patient's body at the desired rate, to stabilize the dosage form against chemical decomposition or mechanical influences. In order to stabilize atorvastatin in the dosage form, it is usually recommended to add a basic substance, calcium carbonate being mentioned as the most preferable one.

Such very low partial pressure of oxygen can be achieved, in industrial practice, either by filling under an inert gas, adapted in accordance with the invention, or by a new technique of packaging under reduced pressure or by use of oxygen absorbers.

The production of blisters is carried out by welding together two sheets. The lower sheet is first shaped in such a manner that a required number of cavities is formed, which cavities correspond in their shape and size to a unit dose of the drug (most often a tablet or capsule). In a further step, the unit dose of the drug is inserted into each of the cavities. The lower sheet, filled in this manner, is overlaid with the upper sheet and both sheets are closely pressed together by means of a pressing roll. In the subsequent step, both sheets are welded together and the welded sheet is cut into individual blisters. A usual method of filling a solid dosage form into the most often used pharmaceutical package, a blister, is carried out in such a manner that the shaped sheet band with tablets passes through a space filled with an inert gas, wherein the upper sheet is pressed against it and welding occurs subsequently. In the atmosphere inside such package there is a-partial pressure of oxygen of 2 - 3 kPa. This state is sufficient with many pharmaceutical substances for their stabilization. However, it cannot prevent oxidation totally and, with especially sensitive substances such as atorvastatin, especially in its amorphous form, it does not ensure complete stability thereof, which leads to the necessity of shortening of the usable life of the composition. Problems of applying the usually employed package under nitrogen with blisters include insufficient wash-out of oxygen from the cavities with tablets, which they entrap with themselves, into the space filled with the inert gas; insufficient tightness of the upper sheet with the lower one immediately before the welding; and possible penetration of air into the cavities immediately before the welding not being avoided in a suitable manner.

The new method of packaging has been developed by optimization of the parameters of the flow of the inert gas and its distribution in accordance with the packaging process. The main feature of this aspect of the invention is introducing the inert gas into the cavities of the lower shaped sheet with such intensity that the content of the gas in the

cavity exchanges at least once, preferably three times. This provision itself can lower the resulting partial pressure of oxygen significantly below 2 kPa, in some cases below 1 kPa. In accordance with a preferred embodiment of the invention, after shaping the cavities in the lower sheet band and filling the same with dosage units, the band enters a purging chamber, constituted by a set of nozzles for targeted introduction of the inert gas into the cavity with the dosage unit and diversion channels for delivery of washed-out air. Air is completely washed out from the cavities by a stream of the inert gas, flowing through the nozzles under a precisely determined and monitored pressure, or flow rate, resp. The flow rate of the inert gas is set in the range of 180 to 3000 l/h. Preferably the flow rate is in the range of 500 to 15001/h. The purging chamber is, together with a wiping station (welding of the upper - cover Al sheet with the lower shaped and filled-in Al sheet), covered in a box with permanent inert atmosphere and a lower pressure above atmospheric than in the purging chamber for sufficient delivery of the washed-out air. In the box there is monitoring of the residual oxygen values with a feedback to the machine run.

As shown below, this method results, under industrial conditions, in a pharmaceutical package with blisters containing a gas with partial pressure of oxygen lower than 0.4 kPa, usually between 0.2 and 0.3 kPa.

In pharmaceutical packaging under reduced pressure of 0.3 - 10 kPa it is more advantageous to use a strip rather than a blister. A strip is a type of packing wherein two sheets are again welded together but none of the sheets is shaped as with the classical blister. The dosage form (a tablet, gelatin capsule, granulate and the like) is inserted into the partially welded strip and not only the filled-in part of the strip, and also the part that will be filled in the subsequent step, are at the same time evacuated, the evacuation taking place for the whole time of the welding operation, wherein complete, air-tight closing of the individual dosage form into the aluminium sheet (strip) occurs.

In the solution by means of oxygen absorbers it is very advantageous if the substances capable of absorbing oxygen are coated on the upper sheet (i.e., the sheet that is not shaped) and separated from the pharmaceutical composition by a permeable membrane: In such a case each dosage unit is protected individually and, after consumption of only a portion of the package, the residue remains protected.

Another solution resides in packaging of the dosage units into blisters, at last one sheet being selected from such a material that is well permeable for oxygen (but preferably poorly permeable for steam). The whole blister is in turn closed in a pouch, in which an absorber is located. It can be for example a polypropylene blister, coated in an A1-A1 pouch. The solution by means of oxygen absorbers has an advantage of technically more simple makeup, wherein it is not necessary to coat the sheet with an absorber.

Surprisingly, it has been shown that there is a close relation between atmospheric oxygen and a suitable formulation. Some formulations, which are relatively successful when storing the dosage form with normal access of oxygen, turn out to be unsuitable for oxygen trapping. On the contrary, those that are less suitable in normal conditions, strengthen the stabilization influence of oxygen trapping. Products, which are in no immediate relation to the oxidation are at fault. The lactone of atorvastatin of the above-mentioned formula II is an example. The basic action of calcium carbonate prevents, in normal conditions, the acid-catalyzed reaction and limits the formation of the lactone (EP 680320). When reducing the amount of oxygen, this effect of calcium carbonate is lowered and the concentration of the lactone increases with time. The use of a base like magnesium oxide or hydroxide is usually considered less suitable. In normal conditions, i.e., with the access of oxygen, the formulation with a magnesium base leads not only to the increase in the amount of usual impurities, but also to the formation of many impurities which are not identified when using calcium carbonate. On the contrary, under reduced partial pressure of oxygen, a base of this type strengthens the stabilization effect described above for the 100% substance. It is again a case of complex stabilization, i.e., not only mere limitation of apparent oxidation products. Therefore, stabilization of atorvastatin by combining the effects of the atmosphere with a partial pressure of oxygen lower than 2 kPa, especially lower than 0.4 kPa, and of magnesium oxide or of packaging under an inert atmosphere, or vacuum, and of magnesium oxide, is considered an especially advantageous embodiment according to this invention.

A further aspect of the invention resides in a suitable analysis of the oxygen content in the pharmaceutical package, without which it would not be possible to carry out the invention. The problem of analysis of the composition of the gas in the pharmaceutical package (especially in blisters) is complicated by necessity to avoid ingestion of atmospheric oxygen from the surroundings when taking a sample, or by lowering the actual oxygen content in the blister in an attempt to avoid this fault when taking a sample.

Three measurement methods have been developed:

1. Measurement A: Measurement of residual oxygen by gas chromatography with collection of gas and manual injection.
Residual oxygen in the inert atmosphere is measured by gas chromatography with a thermal conductivity detector (TCD). The gas is separated in a column containing, as the phase, a molecular sieve, which allows separation of permanent gases. Collection of samples s manual, with readjustment of the blister by deposited septa or by means of dosing from a broken blister by a loop device.
2. Measurement B: Measurement of residual oxygen by means of a microsensor.

Residual oxygen in the inert atmosphere is measured directly in the blister by means of a microsensor, which is situated in a needle. This needle is, through a closed space depleted of oxygen (a chamber purged by nitrogen - measured by this sensor as the background), stuck directly into the individual cells in the blister. The microsensor is based on the suitable instrumental method, which is known as selective for oxygen.

3. Measurement C: Measurement of residual oxygen by means of an in-line set of sensors (in-process control).

Residual oxygen in the inert atmosphere of the machine is measured directly-by means of sensors that are situated in all inertized parts of the packaging machine, including the inlet of nitrogen into the blister cells. These sensors monitor also the outer space.

Obviously, from the point of view of authenticity of the analysis result, methods B and C are more preferable, since no disruption of the blister occurs and possibilities of contamination during analysis are negligible. Moreover, method C unveils very quickly possible faults in industrial packing.

This invention is elucidated in greater detail in the following working examples. These examples are of an illustrative nature.

Examples

Comparative Example 1

Amorphous atorvastatin (1 g) was sealed in a sealable aluminium foil together with the oxygen absorber Ageless® Z100 (Mitsubishi) or with the absorber of oxygen and carbon dioxide Ageless® E100 (Mitsubishi) and the sample was heated at 80 °C for 72 hr. The reference sample was prepared and heated in the same way without the use of absorbers. The results ofHPLC analyses are summarized in Table 4.

Table 4

| Conditions | Total Impurities, % | Lactone of Atorvastatin, % |
|---|---|---|
| Initial | 0.49 | 0 |
| Reference sample | 1.32 | 0.33 |
| Ageless® Z100 | 0.64 | 0.19 |
| Ageless® E100 | 0.60 | 0.14 |

Comparative Example 2

Amorphous atorvastatin (1 g) was sealed in a sealable aluminium foil together with the oxygen absorber Ageless® Z100 (Mitsubishi) or with the absorber of oxygen and carbon dioxide Ageless® E100 (Mitsubishi) and the sample was heated at 40 °C for 1.5 months. The reference sample was prepared and heated in the same way without the use of absorbers. The results of HPLC analyses are summarized in Table 5.

Table 5

| Conditions | Total Impurities, % | Lactone of Atorvastatin, % |
|---|---|---|
| Initial | 0.49 | 0 |
| Reference sample | 1.55 | 0.22 |
| Ageless® Z100 | 0.36 | 0 |
| Ageless® E100 | 0.46 | 0.06 |

Example 3

Tablets having the composition described in Table 6

Table 6

| Composition of the Tablets | Amount, mg |
|---|---|
| calcium salt of atorvastatin | 20.0 |
| lactose monohydrate | 42.0 |
| microcrystalline cellulose | 60.0 |
| calcium carbonate | 88.0 |
| hydroxypropyl cellulose | 30.0 |
| pregelatinized corn starch | 30.0 |
| polysorbate | 1.0 |
| talc | 1.5 |
| sodium salt of crosscarmelose | 6.0 |
| calcium stearate | 0.5 |

were coated with a usual lacquer containing hydroxypropylmethyl cellulose and sealed together with the absorption bag Ageless® Z100 (Mitsubishi) in an aluminium foil and heated at 80 °C for 72 hours. The reference sample was prepared and heated in the same way without the use of oxygen absorber. The results of HPLC analyses are summarized in Table 7.

Table 7

| Conditions | Total Impurities, % | Lactone of Atorvastatin, % |
|---|---|---|
| Initial | 0.60 | 0.03 |
| Reference sample | 4.21 | 2.58. |
| Ageless® Z100 | 1.98 | 1.13 |

Example 4

Tablets containing 20 mg of the amorphous form of calcium salt of atorvastatin having the composition described in Table 8

Table 8

| Composition of the Tablet | Amount, mg |
|---|---|
| calcium salt of atorvastatin | 20.0 |
| lactose monohydrate | 42.0 |
| microcrystalline cellulose | 60.0 |
| calcium carbonate | 88.0 |
| hydroxypropyl cellulose | 30.0 |
| pregelatinized corn starch | 30.0 |
| polysorbate | 1.0 |
| talc | 1.5 |
| sodium salt of crosscarmelose | 6.0 |
| calcium stearate | .0.5 |

were coated with a usual lacquer containing hydroxypropylmethyl cellulose and filled into a glass vial of a volume of 20 ml (reference sample). In the course of other experiments, the oxygen absorber Ageless® Z100 (Mitsubishi), or the absorber of oxygen and carbon dioxide Ageless® E100 (Mitsubishi), or the oxygen absorber Ageless® Z100 (Mitsubishi) and a desiccant were added into the vial besides the tablets. The vials were closed using a HDPE cap and subjected

to 40 °C and 75% RH for 1.5 months. The arrangement of the experiment and the results of HPLC analyses are summarized in Table 9.

Table 9

| Conditions | Total Impurities, % | Lactone of Atorvastatin, % |
|---|---|---|
| Initial | 0.49 | 0 |
| Reference sample | 2.38 | 0.20 |
| Ageless® Z100 | 1.09 | 0.41 |
| Ageless® E100 | 1.10 | 0.48 |
| Ageless® Z100 + desiccant | 0.87 | 0.33 |

Example 5

Tablets containing 20 mg of the amorphous form of calcium salt of atorvastatin having the composition described in Table 10

Table 10

| Composition of the Tablet | Amount, mg |
|---|---|
| calcium salt of atorvastatin | 20.0 |
| lactose monohydrate | 49.6 |
| microcrystalline cellulose | 148.0 |
| magnesium oxide | 14.0 |
| hydroxypropyl cellulose | 28.0 |
| polysorbate | 9.0 |
| sodium salt of crosscarmelose | 9.0 |
| magnesium stearate | 1.4 |
| silicon dioxide | 1.0 |

were coated with a usual lacquer containing hydroxypropylmethyl cellulose, filled into a glass vial of a volume of 20 ml closed with a HDPP cap together with the absorption bag Ageless® Z100 (Mitsubishi) and stored at 40 °C and 75% RH for 1.5 months. A reference sample was prepared in the same way without adding the oxygen absorber. A preliminary analysis found the partial pressure of oxygen after completion of the experiment. It has shown that the pressure in the vial atmosphere dropped to 0.3 kPa. The results of HPLC analyses are summarized in the following table (Table 11).

Table 11

| Conditions | Total Impurities, % | Lactone of Atorvastatin, % |
|---|---|---|
| Initial | 0.79 | 0 |
| Reference sample | 2.12 | 0 |
| Ageless® Z100 | 0.84 | 0 |

Example 6

In order to find out influence of antioxidants on stability of the dosage form, mixtures of the amorphous form of atorvastatin with a basic component and antioxidants were made. These mixtures were filled into glass vials of a volume of 20 ml, closed using a HDPE cap, and heated at 40 °C and 75% RH for 6 weeks. The composition of the said mixtures and the results of HPLC, analyses are summarized in Table 12.

Table 12

| Mixture Number | Mixture Composition | Component Ratios | Total Impurities, % | Lactone of Atorvastatin, % |
|---|---|---|---|---|
| 2 | calcium salt of atorvastatin | 1 | 2.03 | 0.05 |
| | calcium carbonate | 3 | | |
| 5 | calcium salt of atorvastatin | 1 | 4.04 | 1.12 |
| | calcium carbonate | 3 | | |
| | vitamin E | 1 | | |
| 6 | calcium salt of atorvastatin | 1 | 4.53 | 1.58 |
| | calcium carbonate | 3 | | |
| | β-carotene | 1 | | |
| 9 | calcium salt of atorvastatin | 1 | 4.71 | 1.29 |
| | calcium carbonate | 3 | | |
| | sodium ascorbate | 1 | | |

It follows from the above experiment that the use of antioxidants, routinely used in the pharmaceutical industry, does not prevent oxidation processes in the dosage form.

Example 7. Test of stability under reduced pressure.

A pure amorphous atorvastatin substance was close din a tempered box under air pressure of 5.5 - 7.5 kPa. The partial pressure of oxygen was estimated as 1.2 -1.5 kPa.

The measurement has given the following results:

Table 13

| Sample | Storage Time, days/hrs | Storage Temperature, °C | Total Impurities, % |
|---|---|---|---|
| 0010903/12 | 23 days | 20-25 | 0.29 |
| 0010903/15 | 42 days | 40-42 | 0.35 |
| 0010903/17 | 55 hrs | 60-65 | 0.33 |
| 0010903/18 | 160 hrs | 80-82 | 0.37 |

For comparison, sample 0010903/18 was kept in a thermostat at 80°C under normal atmospheric pressure, i.e. partial pressure of oxygen about 18 kPa for 160 hours. The total impurities then reached 1.92 %.

Example 8. Packaging of the dosage form under nitrogen

Tablets containing 40 mg of the amorphous form of the calcium salt of atorvastatin having a composition proportional to that of Example 5 were packaged in an industrial packaging line adapted for packaging under nitrogen. The commercially produced blistering machine WinPack TR 130 from Italian company IMA was used for the packaging. The shaped lower sheet, containing the finished tablets, was transported into a purging chamber, into which also nitrogen under a pressure of 0,1 MPa above atmospheric at the outlet of the nitrogen source has been introduced. The flow rate of the gas in the chamber, i.e. the inlet of nitrogen and its outlet with admixture of air, was maintained at 1500 l/h. The pressure above atmospheric of nitrogen in the protective box was approximately 10 kPa. The tablets were packaged into aluminium A1/A1 blisters. The finished package was subjected to a standard stability test; subjected to a load of 40

°C and 75% RH for 3 months. The partial pressure of oxygen in the aluminium package was tested after packaging and after 3 months of storage. Results of HPLC analyses are summarized in the following table.

Measurement of residual oxygen in the blister was made by a gas chromatography method with collecting the gas and with manual injection. Collection of the gas was made in three different positions in the blister.

Table 14

| Sample Designation | Time of Load, months | Total Impurities, % | $P_O$, kPa | | |
|---|---|---|---|---|---|
| | | | Position 1 | Position 2 | Position 3 |
| 040903 | 0 | 0.54 | 0.24 | 0.23 | 0.24 |
| | 3 | 0.50 | 0.25 | 0.27 | 0.26 |
| 070903 | 0 | 0.76 | 0.20 | 0.19 | 0.20 |
| | 3 | 0.50 | 0.19 | 0.20 | 0.18 |

Example 9. Packaging of the dosage form under reduced pressure

Tablets containing 40 mg of the amorphous form of the calcium salt of atorvastatin having a composition proportional to that of Example 5 were closed in an experimental apparatus by welding into an Al-Al sheet (strip model) in air atmosphere under reduced pressure 1 - 1.4 kPa, i.e., under a partial pressure of oxygen of about 0.18 - 0.25 kPa, and subjected to a load of 40°C and 75% RH for 3 months. The partial pressure of oxygen in the aluminium package was deduced from the total pressure and usual air atmosphere composition. Results of HPLC analyses are summarized in the following table.

Table 15

| Sample Designation | Time of Load, months | Total Impurities, % |
|---|---|---|
| 040903 | 0 | 0.54 |
| | 3 | 0.55 |
| 070903 | 0 | 0.76 |
| | 3 | 0.50 |

**Claims**

1. A method for the stabilization of the pharmaceutical active solid substance atorvastatin embedded in a gaseous mixture **characterized in that** a drug in the form of tablets or capsules containing atorvastatin in an amount of 1 to 60 % by weight, packaged in a blister is stabilized and in the surrounding gaseous mixture a partial pressure of oxygen of at most 2 kPa is maintained.

2. The method according to claim 1 **characterized in that** the partial pressure of oxygen is maintained lower than 1 kPa.

3. The method according to claim 1 **characterized in that** the partial pressure of oxygen is maintained lower than 0.4 kPa.

4. The method according to claims 1-3 **characterized in that** atorvastatin is in a mixture containing solid magnesium oxide in an amount of 0.1 to 50 % by weight.

5. The method according to any of the preceding claims **characterized in that** atorvastatin is predominantly in an amorphous form.

6. The method according to claim 1 **characterized in that** the blister is an aluminium blister of the Al-Al type.

7. The method according to claim 1 **characterized in that** the drug is packaged in a polypropylene blister, which is further enveloped in an Al-Al pouch.

8. The method according to claim 1 **characterized in that** the drug is packaged in a strip.

9. The method according to any of the preceding claims **characterized in that** the said partial pressure is achieved by use of at least one oxygen absorber.

10. The method according to claim 9 **characterized in that** the oxygen absorber is selected from the group including a humidity-activated oxygen absorber, a self-activating absorber, an ultraviolet-radiation-activated absorber, a radiation-activated absorber, a microwaves-activated absorber, an absorber activated by a combination of activation processes, or an absorber without necessity of activation.

11. The method according to claim 10 **characterized in that** the oxygen absorber is a self-activating absorber.

12. The method according to any of claims 1-8 **characterized in that** the said partial pressure is achieved by use of excess of an inert gas.

13. The method according to any of claims 1, 6, 7 and 12 **characterized in that** the said partial pressure is achieved by packaging in a blister-forming machine, by introducing a stream of an inert gas, preferably nitrogen, into cavities in the lower shaped sheet with such intensity that the content of the gas in the cavity exchanges at least once, preferably three times.

14. The method of claim 13 **characterized in that** the flow rate of the inert gas ranges from 180 to 3000 l/h.

15. The method of claim 14 **characterized in that** the flow rate of the inert gas ranges from 500 to 1500 l/h.

16. The method according to any of claims 13-15 **characterized in that** the band with shaped cavities is brought into a purging chamber, consisting of a set of nozzles, destined for targeted introduction of the inert gas to the cavities, and of diversion channels for the washed-out air outlet, the purging chamber being located in a box having permanently inert atmosphere, wherein, subsequently, an upper covering band is pressed against said band with the cavities and, finally, the blister is welded together.

17. The method according to claim 13 **characterized in that** the flow rate of the inert gas into the purging chamber is maintained at 1300 - 15001/h.

18. The method according to any of claims 1-8 **characterized in that** the said partial pressure is achieved by packaging under a pressure of 0.3 to 10 kPa.

19. A pharmaceutical composition in a pharmaceutically suitable packing comprising a blister, obtainable according to claim 17, surrounded with a gaseous mixture constituted by the inert gas fed during the packaging, **characterized by** a partial pressure of oxygen lower than 1 kPa.

20. The pharmaceutical composition according to claim 19, **characterized by** a partial pressure of oxygen lower than 0.4 kPa.

**Patentansprüche**

1. Verfahren zur Stabilisierung des pharmazeutisch wirksamen festen Stoffes Atorvastatin, eingelagert in einer Gasmischung, **dadurch gekennzeichnet, dass** man ein Heilmittel in der Tablett- oder Kapselform, enthaltend atorvastatin in die Menge von 1 bis 60 Gew.-%, verpackt in einem Blister, stabilisiert, und in der Umgebungsgasmischung ein Sauerstoffpartialdruck von maximal 2 kPa erhalten wird.

2. Verfahren gemäß Anspruch 1, **dadurch gekennzeichnet, dass** der Sauerstoffpartialdruck unter 1 kPa erhalten wird.

3. Verfahren gemäß Anspruch 1, **dadurch gekennzeichnet, dass** der Sauerstoffpartialdruck unter 0,4 kPa erhalten wird.

4. Verfahren gemäß Ansprüche 1 bis 3, **dadurch gekennzeichnet, dass** Atorvastatin in einer Mischung, enthaltend festes Magnesiumoxid in der Menge von 0,1 bis 50 Gew.-%, vorliegt.

5. Verfahren gemäß einem der vorhergehenden Ansprüche, **dadurch gekennzeichnet, dass** Atorvastatin in überwiegend amorpher Form ist.

6. Verfahren gemäß Anspruch 1, **dadurch gekennzeichnet, dass** es sich beim Blister um einen Aluminiumblister des Typen Al-Al handelt.

7. Verfahren gemäß Anspruch 1, **dadurch gekennzeichnet, dass** das Heilmittel im Polypropylenblister verpackt ist, welcher weiter in einem Al-Al-Beutel eingeschlagen ist.

8. Verfahren gemäß Anspruch 1, **dadurch gekennzeichnet, dass** das Heilmittel in einer Streifenpackung verpackt ist.

9. Verfahren gemäß einem der vorhergehenden Ansprüche, **dadurch gekennzeichnet, dass** der genannte Partialdruck durch Einsatz mindestens eines Sauerstoffabsorbers erreicht wird.

10. Verfahren gemäß Anspruch 9, **dadurch gekennzeichnet, dass** der Sauerstoffabsorber aus der Gruppe der Sauerstoffabsorber, aktiviert durch Feuchtigkeit, der selbstaktivierenden Absorber, Absorber aktiviert durch ultraviolette Strahlung, Absorber aktiviert durch Radiation, Absorber aktiviert durch Mikrowellen, Absorber aktiviert durch einer Kombination mehrerer Aktivierungverfahren, ggf. Absorber ohne Aktivierungsnötigkeit, ausgewählt wird.

11. Verfahren gemäß Anspruch 10, **dadurch gekennzeichnet, dass** der Sauerstoffabsorber ein selbstaktivierender Absorber ist.

12. Verfahren gemäß einem der Ansprüche 1 bis 8, **dadurch gekennzeichnet, dass** der genannte Partialdruck mit Hilfe eines Überflusses an Inertgas erreicht wird.

13. Verfahren gemäß einem Ansprüche 1, 6, 7 und 12, **dadurch gekennzeichnet, dass** der genannte Partialdruck durch Verpacken auf der Blisterpackmaschine unter Zufuhr eines Stromes von Inertgas, insbesondere Stickstoff, in die Hohlräume der unteren geformten Folie erreicht wird, mit solcher Intensität, dass der Gasanteil im Hohlraum mindestens einmal, vorteilhafter dreimal ausgetauscht wird.

14. Verfahren gemäß Anspruch 13, **dadurch gekennzeichnet, dass** die Durchflussrate des Inertgases zwischen 180 und 3000 l/h beträgt.

15. Verfahren gemäß Anspruch 14, **dadurch gekennzeichnet, dass** die Durchflussrate des Inertgases zwischen 500 und 1500 l/h beträgt.

16. Verfahren gemäß einem der Ansprüche 13 bis 15, **dadurch gekennzeichnet, dass** das Band mit den geformten Hohlräumen in eine Auswaschkammer eingeleitet wird, bestehend aus einer Zusammenstellung von Düsen, bestimmt zu gezielter Zufuhr des Inertgases zu den Hohlräumen, und aus Abfuhrkanälen für Abführung der ausgewaschenen Luft, wobei die Auswaschkammer in einer Box mit ständiger inerten Atmosphäre platziert ist, wo nachfolgend zum Band mit den Hohlräumen das obere Abdeckband zugeführt wird und der Blister abschließend verschweißt wird.

17. Verfahren gemäß Anspruch 13, **dadurch gekennzeichnet, dass** die Durchflussrate des Inertgases in die Auswaschkammer auf 1300 bis zu 15001/h erhalten wird.

18. Verfahren gemäß einem der Ansprüche 1 bis 8, **dadurch gekennzeichnet, dass** der genannte Partialdruck durch Verpacken unter einem Druck von 0,3 bis 10 kPa erreicht wird.

19. Pharmazeutische Zusammensetzung in einer pharmazeutisch geeigneten Verpackung, beinhaltend einen Blister, herstellbar gemäß Anspruch 17, umgeben von einer Gasmischung, erstellt durch das im Verlauf der Verpackung zugeführte Inertgas, **gekennzeichnet durch** den Sauerstoffpartialdruck von unter 1 kPa.

20. Pharmazeutische Zusammensetzung gemäß Anspruch 19, **gekennzeichnet durch** den Sauerstoffpartialdruck von unter 0,4 kPa.

## Revendications

1. Procédé de stabilisation de la substance solide pharmaceutique active atorvastatine, déposée en un mélange gazeux, **caractérisé par** ce que l'on stabilise un médicament sous forme de comprimés ou capsules contenant l'atorvastatine dans une quantité de 1 à 60 % en poids, emballé dans un blister, et que l'on maintient dans le mélange gazeux environnant une pression partielle d'oxygène maximal de 2 kPa.

2. Procédé selon la revendication 1, **caractérisé en ce que** la pression partielle d'oxygène est maintenue en dessous de 1 kPa.

3. Procédé selon la revendication 1, **caractérisé en ce que** la pression partielle d'oxygène est maintenue en dessous de 0,4 kPa.

4. Procédé selon les revendications 1 à 3, **caractérisé en ce que** l'atorvastatine est dans un mélange comportant de l'oxyde de magnésium solide dans une quantité de 0,1 à 50 % en volume.

5. Procédé selon l'une quelconque des revendications précédentes, **caractérisé en ce que** l'atorvastatine est à prépondérance sous forme amorphe.

6. Procédé selon la revendication 1, **caractérisé en ce que** le blister est un blister en aluminium de type A1-A1.

7. Procédé selon la revendication 1, **caractérisé en ce que** le médicament est emballé dans un blister en polypropylène, lui-même emballé dans un pouch Al-Al.

8. Procédé selon la revendication 1, **caractérisé en ce que** le médicament est emballé dans un strip.

9. Procédé selon l'une quelconque des revendications précédentes, **caractérisé en ce que** ladite pression partielle est obtenue par l'utilisation d'au moins un absorbeur d'oxygène.

10. Procédé selon la revendication 9, **caractérisé en ce que** l'absorbeur d'oxygène est choisi parmi le groupe comportant un absorbeur d'oxygène activé par l'humidité, d'un absorbeur autoactivant, d'un absorbeur activé par les rayons ultra-violet, d'un absorbeur activé par des radiations, d'un absorbeur activé par les micro-ondes, d'un absorbeur activé par une combination de processus activants ou d'un absorbeur sans nécessité d'activation.

11. Procédé selon la revendication 10, **caractérisé en ce que** l'absorbeur d'oxygène est un absorbeur autoactivant.

12. Procédé selon l'une quelconque des revendications 1 à 8, **caractérisé en ce que** ladite pression partielle est obtenue à l'aide d'un excès de gaz inerte.

13. Procédé selon l'une quelconque des revendications 1, 6, 7 et 12, **caractérisé en ce que** ladite pression partielle est obtenu par l'emballage sur un appareil de blister introduisant un courant de gaz inerte, d'azote de préférence, dans les cavités dans la feuille profilée inférieure avec l'intensité telle que le contenu de gaz dans les cavités soit changé au moins une fois et de préférence trois fois.

14. Procédé selon la revendication 13, **caractérisé en ce que** le débit de gaz inerte varie de 180 à 3000 1/h.

15. Procédé selon la revendication 14, **caractérisé en ce que** le débit de gaz inerte varie de 500 à 1 500 l/h.

16. Procédé selon l'une quelconque des revendications 13 à 15, **caractérisé en ce que** l'on introduit la bande avec des cavités formées dans une chambre de rinçage, constituée d'un ensemble de tuyères, destinées à l'arrivée visée du gaz inerte dans les cavités, et des canaux d'évacuation pour l'évacuation de l'air rincé, la chambre de rinçage étant située dans une boîte à atmosphère inerte permanente, où la bande supérieure de couverture est par la suite pressée sur la bande aux cavités et où enfin le blister est soudé.

17. Procédé selon la revendication 13, **caractérisé en ce que** le débit de gaz inerte dans la chambre de rinçage est maintenu entre 1300 et 1500 l/h.

18. Procédé selon l'une quelconque des revendications 1 à 8, **caractérisé en ce que** ladite pression partielle est

obtenue par l'emballage sous une pression de 0,3 à 10 kPa.

**19.** Composition pharmaceutique dans un emballage pharmaceutiquement approprié, comportant un blister, obtenable suivant la revendication 17, entouré d'un mélange gazeux, constitué par le gaz inerte introduit au cours de l'emballage, **caractérisée par** une pression partielle d'oxygène inférieure à 1 kPa.

**20.** Composition pharmaceutique selon la revendication 19, **caractérisée par** une pression partielle d'oxygène inférieure à 0,4 kPa.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4681893 A
- US 5273995 A
- US 5969156 A
- US 6121461 A
- WO 03004470 A
- WO 0136384 A
- US 6087511 A
- EP 680320 A
- WO 0034525 A
- WO 0176566 A
- WO 0193859 A
- WO 02072073 A
- US 20020132359 A
- WO 2004032920 A


### Non-patent literature cited in the description


- **YOSHIKAWA, Y. ; AMEMIYA, A. ; KOMATSU, T. ; INOUE, Y. ; YUYAMA, M.** Oxygen Absorbent for Food Packaging. *Jpn. Kokai Tokkyo Koho,* 1978, 56-33980
- **STEPHEN R. BYRN.** Solid State Chemistry of Drugs. Academic Press, 1982
- *Tetrahedron,* 1993, vol. 49 (10), 1979-1984
- *Pharmaceutical Development and Technology,* 2002, vol. 7 (1), 1-32
- **K.C. WATERMAN ; M.C. ROY.** *Pharmaceutical Development and Technology,* 2002, vol. 7 (2), 227-234
- **VINOD DANIEL ; FRANK L. LAMBERT.** *Waac Newsletter,* 1993, vol. 15 (2), 12-14